# EUROPEAN PATENT SPECIFICATION

(11) **EP 1 138 782 B1**
(45) Date of publication and mention of the grant of the patent: **20.10.2010**
(21) Application number: 01106974.7
(22) Date of filing: 21.03.2001
(51) Int. Cl.: C12Q 1/68

(54) **Multiplex sequence variation analysis of DNA samples by mass spectrometry**
Multiplex Sequenzvariationsanalyse von DNA-Proben mittels Massenspektrometrie
Variation multiplex de séquence pour l'analyse des échantillons d'ADN par spectrométrie de masse

(30) Priority: 26.03.2000 DE 10015797
(43) Date of publication of application: 04.10.2001
(73) Proprietor: Bruker Daltonik GmbH, 28359 Bremen (DE)
(72) Inventor: Jäschke, Andres, 14109 Berlin (DE); Hausch, Felix, Stanford, CA 94305 (US)

(56) References cited:
- EP-A- 1 234 888
- WO-A-99/02728
- US-A- 5 872 003
- GRIFFIN T J ET AL: "Single-nucleotide polymorphism analysis by MALDI-TOF mass spectrometry" TRENDS IN BIOTECHNOLOGY, ELSEVIER, AMSTERDAM, NL, vol. 18, no. 2, February 2000 (2000-02), pages 77-84, XP004187253 ISSN: 0167-7799

## Description

### Field of invention

The invention relates to the simultaneous investigation of variations of distinct nucleic acid sequences within a complex nucleic acid mixture of a DNA sample by mass spectrometry.

### Prior art

With the continuously increasing amount of sequence information from the various sequencing projects currently being undertaken, and the growing number of completely elucidated genomes, genetic studies have shifted more towards reproducible or secondary sequence analysis based on already known sequences. Currently the genomes from over 25 organisms have been completely elucidated, and the human genome as well as many others will be added to this list in the coming years. The information obtained in this way can be used repeatedly and directly for analyzing specific characteristics, e.g. for detecting sequences which are specific for disease pathogens. Genetically determined diseases or disease predispositions can often be attributed to specific mutated gene sequences. Human medicine is currently aware of more than 3000 monogenic diseases induced by alterations in single genes. Sometimes a pathogenic characteristic can be attributed to a mutation of a single nucleotide. During the course of the Human Genome Project (HUGO) this number will increase further and will indeed be extended by multigenic factors.

The growing number of relevant gene markers sets new standards for nucleic acid diagnostics. Classical sequencing by the Sanger procedure, employed for the original sequencing of the currently elucidated genomes, is unsuitable here since it is time-consuming and can not be completely automated.

With the development of gentle mass spectroscopic procedures which permit the analysis of large, intact biomolecules, a highly promising alternative has now become available. In this way, MALDI TOF MS (MALDI = Matrix Assisted Laser Desorption/Ionization; TOF = Time-Of-Flight; MS = mass spectrometry) can directly determine the molecular weight of nucleic acids (Hillenkamp and Karas, 1990, Methods in Enzymology, 280). Unlike classical Sanger sequencing by PAGE (PolyAcrylamide Gel Electrophoresis), the sequence-specific fragment ladder of a DNA sample is reported with a single mass value. This technique has already been successfully employed to sequence the human p53 gene (Fu et aL 1998, Nat. BiotechnoL, 381), and in US 5,777,324 (F. Hillenkamp, 1996) a promising device has been put forward for this purpose. Through improvement of the measuring conditions (gentler excitation in the IR range, neutral matrix), it has become possible recently to measure much larger DNA molecules with over 2,500 nucleotides (Berkenkamp et aL, 1998, Science, 260). As a result the reading and measurement accuracy with mass spectrometric sequencing should markedly improve (WO 99/57318). In this context, one should also mention the importance of using stable or isotopically pure nucleotide analogs (WO 96/27681, I. Gut et aL). A procedure for a more precise mass spectroscopic DNA sequencing through the use of internal calibration is put forward in WO 95/14108 (M.A. Reeve et al.).

The diagnostics of already known genes has been substantially simplified by the development of so-called DNA chips (Gerhold et al. 1999, Trends in Biochemical Sciences, 168-173). On such a chip, surface areas are functionalized with oligonucleotides that are complementary to the genetic material to be analyzed. As a rule, oligonucleotide markers are organized in an array so that hybridization at a particular position can confirm the presence of a corresponding sequence within the biological sample (Fodor, 1997, Science, 393-395). The evaluation of the hybridization test is usually performed by fluorescence marking of the samples and scanning of the DNA chip. The major advantage of DNA analysis using DNA chips is the high throughput; i.e. on a 1.6 x 1.6 cm chip up to 137,000 oligonucleotides can be placed and read within 10 minutes (Chee et aL 1996, Science, 610-614). The fluorescence analysis of the hybridized samples, however, does not permit any further acquisition of information regarding the target sequence. Furthermore, the hybridization efficiency is dependent on the base composition and is significantly influenced by secondary structures; as a result, only relative statements can be made usually (i.e. comparisons with internal standards). Apart from that, individual mismatches are tolerated, and these can only be excluded by extensive control experiments.

The mass spectrometric evaluation of DNA chips offers the general benefit that apart from providing information about the presence of investigated nucleic acid sequences, additional information can be obtained regarding their molecular weight. This third dimension of measurement (as a supplement to the two dimensional localization on the chip) can be introduced for internally controlling the hybridized DNA probes. With mass spectroscopic DNA sequencing this has already been exploited elsewhere for an extra controlling of the measured nucleic acid fragments and interpreting the observed sequence ladder (Kirpekar et al., 1998, Nucleic Acids Research, 2554-2559).

Since with MALDI TOF MS measurements the nucleic acid fragments are presented on a flat surface, this method is particularly suitable for the rapid evaluation of nucleic acid chips. The nucleic acids to be measured are first enclosed in a solid, organic matrix, which is then desorbed from the surface by a pulse of laser light. In this process isolated nucleic acids are carried away as a matrix cluster into the gas phases, ionized by the dissolving organic phase and then detected by their characteristic time of flight in the electrical field. With the laser stimulation, individual points within the DNA arrays can be evaluated in a targeted fashion. Such a concept is described in WO 94/16101 (H. Köster) whereby the samples to be investigated are immobilized on an appropriate surface and then incubated with an oligonucleotide marker. Such probes are if necessary sequence-specifically modified before they are detected by MALDI TOF mass spectrometry. This requires a separate purification and targeted positioning for each of the DNA samples to be investigated. Because of the sheer numbers of samples to be studied - e.g. all 3000 important point mutations currently known - a considerable effort is involved in the analysis. For this reason nucleic acid modifications are recommended which simplify a covalent immobilization of the samples onto the chip (WO 98/20020, O'Donnell et al.). Photocleavable residues have also been described as supplementary modifications for MALDI TOF measurements.(Olejnik et al., 1998, Nucleic Acids Research, 3572-3576), which enable a light-regulated release of samples. The use of photocleavable sites was similarly suggested in WO 98/20166 (Köster et al.), so that detection reactions could be carried out individually for each sample and that these reaction mixtures could be spotted in an array format on a MALDI TOF compatible chip. In this way a functional group on the nucleic acid probe allows a specific purification on a specially prepared surface while the photocleavable sites enable then a photolytic release for MALDI TOF MS detection. The use of lysable primers as DNA probes for mass spectroscopic sequencing is suggested also in WO 96/37630 (J.A. Montforte et al.)., whereby these should lead to a shortening of the measured nucleic acid fragments and thereby to an improved readability. The application of immobilized primers in a two-step amplification process is described in DE 19710166 (Gut, I. and Franzen J., 1998) whereby the automation and highly parallel processing of nucleic acids is promoted by binding to magnetic particles.

In general, covalently immobilized oligonucleotide probes convey the benefit of an improved and easier handling. In this way the most often varied reaction conditions including denaturing, washing, or temperature changes are enabled, i.e. conditions necessary for solid phase enzyme reactions in particular. Covalently immobilized probes are not, however, accessible for MALDI TOF analysis since they can not be desorbed from the surface substrate.

The detection of nucleic acids by hybridization offers the unique opportunity, consistent with the Watson Crick base pairing rule, to generate complementary oligonucleotide probes for any target sequence. At least from a biological standpoint, such an interaction is relatively nonspecific. Sporadic mismatches are tolerated or can be overlain by other effects such as unusually stable secondary structures (e.g. those with a high G-C composition). However, when sequencing or detecting mutations of single, critical nucleotides (SNP = Single Nucleotide Polymorphism), a reproducible and reliable resolution of single bases is crucial. Enzymatic transformations are clearly superior here to simple hybridization. Restriction enzymes only cut a recognition sequence at a defined position dependent on the methylation pattern. Endonucleases chiefly hydrolyze single nucleotides and DNA ligases are very fastidious about perfect base-pairing at the ligation site. DNA polymerases often possess an additional proof reading activity which can correct their own mistakes. This principle is utilized with classical DNA detection procedures such as polymerase chain reaction (PCR), ligase chain reaction (LCR), RNA footprinting or Sanger sequencing.

Regarding the detection of immobilized nucleic acids, enzymatic modifications have also been recommended for specifically detecting individual nucleotides (GB 2308188, Minter, S.J., 1997). In WO 98/20166 (Köster et al.), detection of modified oligonucleotide probes is performed by mass spectrometry. In both cases the target sequence to be analyzed is immobilized on the solid phase, so that these have to be applied to the surface before each detection. If the oligonucleotide probe is immobilized, this occurs after modification so that purification is made easier.

With increasing knowledge of the genetic factors responsible for particular predispositions, diseases or health risks, the number of relevant nucleotides to be tested within a biological sample has also grown. With enzymatic detection this classically requires one reaction and detection per target sequence. Even with automation and parallel sample processing with an extremely high throughput, this requires considerable preparation.

By using combinations of processes, many similar reactions can be performed parallelly in a single reaction step. Thus, with multiplex PCR, various DNA segments from a biological sample can be simultaneously amplified in a single reaction mixture through addition of a range of primer pairs. In any such reaction mixture, the problem is then usually shifted to find separate detection methods to unravel the complex sequence mixture. In order to suppress the enrichment of unwanted nucleic acids especially when studying severely underrepresented sequences, a nested PCR procedure can be used. With this technique the specificity is doubled in a two-step amplification by using two primer pairs boxed into each other.

### Objective of the invention

The invention should combine, for the investigation of variations in preselected DNA sequences, the highly parallel sample throughput of oligonucleotide chips, the detection specificity of template-dependent modifications, and the high amount of information associated with mass spectrometric detection, in a multiplex analysis with the assistance of photolytically cleavable oligonucleotide probes.

A main objective of the invention is to check a set of relevant (and preferably all) point, insertion, or deletion mutations in a patient in a single reaction step.

### Summary of the invention

It is a basic idea of the invention to use spatially separated, photocleavable oligonucleotide probes on a chip, and to perform multiplex sequence-dependent modification of the oligonucleotide probes, enabling the mass spectrometric detection of the target sequence variations by measuring the masses of the modified, detached probes directly on the chip, whereby the complex target sequence mixture is spatially separated due to the defined positions of the oligonucleotide probes on the chip.

To increase the specificity and to enable correct detection of target sequence variations, the oligonucleotide probes should be enzymatically modified dependent on the particular target sequences. Covalent immobilization of the oligonucleotide probes allows to undertake the required processes and reactions without muddling the probes. The processes and reactions are performed particularly by temperature steps for hybridizing the target sequences on the probes and for subsequent enzymatic modifications, by denaturing solvents for efficient washing and separation of contaminating traces of nucleic acids, and by extreme pH conditions during uptake into the MALDI TOF matrix. Because of the potential for acquiring extra information, detection is performed by mass spectrometry (particularly by MALDI TOF MS), which is enabled by photolytically susceptible cleavage sites on the oligonucleotide probes.

The photocleavable oligonucleotide probes include nucleic acids 5 to 100 nucleotides long (usually 20 to 25 nucleotides long) consisting of DNA, RNA, PNA, or their derivatives, which are complementary to target sequences and which hybridize to the nucleotides under investigation either at their ends, in the middle, or exactly in position. Photocleavable oligonucleotide probes also contain a photolytically susceptible site which can be selectively cleaved by the influence of light, usually o-nitrobenzyl groups. Target sequences as understood in the sense of this invention are genetic zones which might contain relevant mutations, e.g. single nucleotide polymorphisms (SNPs). Template-dependent modifications are transformations, usually enzymatic transformations, of the oligonucleotide probes which only occur upon hybridization of a specific sequence, so that by modifying the probes one can obtain information about details of the target sequence. Oligonucleotide arrays as understood in this invention are surfaces (usually on chips) with 10 to 100000 (typically 100 to 1000) spatially separated locations whereby each location is functionalized with a specific type of oligonucleotide probe.

Another basic idea of the invention is that the template-dependent modification of the probes and the mass spectroscopic detection should take place on the same surface. Hence, photolytic release from the solid substrate and desorption from the matrix can occur simultaneously by a laser pulse so that individual positions of the photocleavable oligonucleotide chips can be read one after the other, preferably in a photolytic manner.

It is characteristic for the invention that the surface fixing of the photocleavable probes occurs before the probe modification for detection, and that the immobilized oligonucleotide probes rather than the hybridized target sequences are enzymatically modified.

Template-controlled modification of oligonucleotide probes is understood as enzymatic alteration dependent on a hybridized target sequence, whereby such changes can later provide information about the nucleotide composition of the target sequence. The invention is partially based upon enzymatic modifications accomplished by a template-dependent primer elongation of the oligonucleotide probes. This explicitly includes point mutation analysis or chain terminating sequencing using dideoxynucleotide triphosphates.

Another basic idea of the invention is to achieve enzymatic detection by template-dependent DNA ligation, whereby the reporter oligonucleotides to be ligated are added to the detection reaction as a combined mixture.

Reporter nucleotides are nucleic acids 5 to 100 nucleotides long - chiefly 20 to 25 nucleotides long - primarily consisting of DNA, RNA, PNA, or their derivatives, which are complementary to a section adjacent to the immobilized oligonucleotide probes on the appropriate target sequence. Only with perfect base pairing at the interface between the oligonucleotide probes and reporters will DNA ligation occur, and because of this the base composition of the target sequence at the interface can be determined. The invention is designed so that the sequence specificity of the detection is raised due to the additional hybridization of the reporter oligonucleotides, and that especially insertion and deletion mutations can be detected in this way. A further idea of the invention is that the reporter oligonucleotides should carry an additional recognition group, permitting a simplified or alternative detection. Such groups might include mass, fluorescence, or affinity markers or even another light sensitive group. The reporter oligonucleotides can also contain stabilized or neutralized nucleotides for a more efficient mass spectroscopic detection.

Another basic idea of the invention is to determine the composition of the target sequence by template-dependent nucleotide cleavage of the oligonucleotide probes. This includes especially a template-dependent restriction digest, whereby the methylation pattern of the target sequences in particular can be reported. For this purpose a combined mixture of different restriction enzymes is the preferred method of choice. The invention uses endonucleases which cleave with a perfect base pairing or only a partial degree of mismatching. A preferred procedure involves oligonucleotide probes with a single, internal ribonucleotide, which can only be hydrolyzed for example by RNase H with perfect pairing to the investigated DNA counterstrand.

A main objective of the invention is to check a set of relevant (and preferably all) point, insertion, or deletion mutations in a patient in a single reaction step. This involves a pre-amplification of the relevant preselected target sequence sections by multiplex PCR using external primer pairs, a multiplex analysis by ligation of reporter oligonucleotides to photocleavable sites, as well as photolytic reading of the ligation products by a subsequent MALDI TOF detection. Thus, oligonucleotide reporters and probes explicitly lie within sequences enclosed by the external primers. Another main implementation of the invention involves the multiplex resequencing of already known genomes for the purpose of reporting clinically relevant mutations.

### Brief description of the figures:

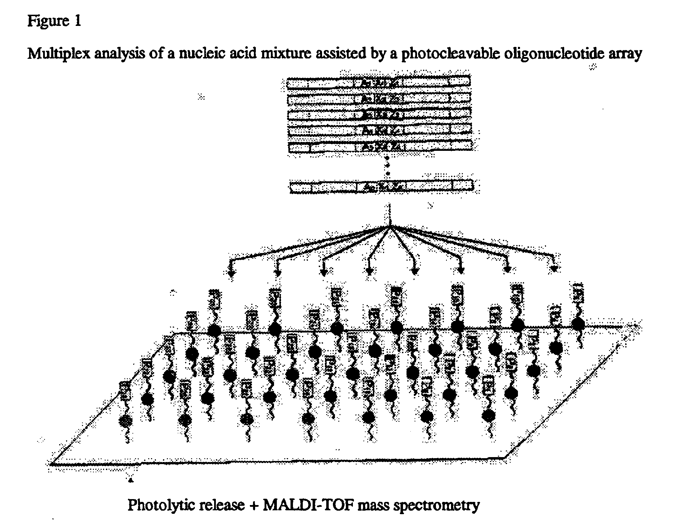

Figure 1 describes the ensemble of target sequences which hybridize at the appropriate positions of the corresponding complementary oligonucleotide probes. P₁₋ₙ. X₁₋ₙ symbolizes the nucleotides to be checked in the multiplex analysis for relevant mutations. A₁₋ₙ and Z₁₋ₙ represent adjacent sequences which are introduced for positioning on the oligonucleotide chip and for the enzymatic detection reactions. The large parallelogram represents the surface of the chip upon which the oligonucleotide probes are immobilized covalently via the spacers depicted as wavy lines. The black circles correspond to the photocleavable sites which permit a targeted release in response to a laser pulse with simultaneous detection of the modified oligonucleotide probes by MALDI TOF mass spectrometry.

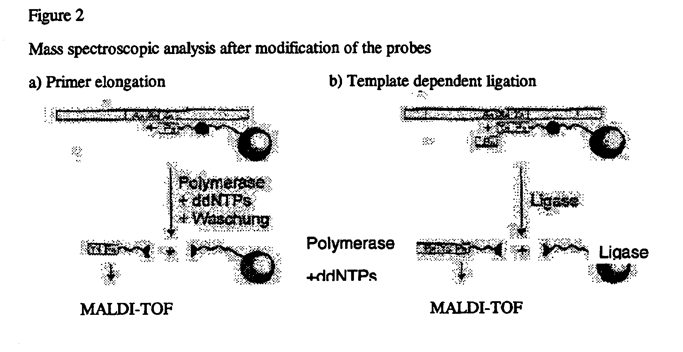

Figure 2 illustrates two methods for enzymatic detection involving template-dependent modification of the photocleavable oligonucleotide probes.
a) The partial sequence Zₙ of an exemplary target sequence hybridizes with a complementary oligonucleotide probe Pₙ. which is immobilized onto the solid substrate via a photocleavable linker depicted in the diagram as a wavy line (shown as a sphere). This is then elongated according to the tested nucleotide Xₙ by a DNA polymerase to form the complementary dideoxynucleotide Yₙ. The modified oligonucleotide probe YₙPₙ is then photolytically released at the photocleavage site (black circle) and measured by MALDI TOF spectrometry.
b) Insertion, deletion or point mutation analysis by template-dependent DNA ligation: the sequence excerpt XₙZₙ of a target sequence deposits itself on the immobilized oligonucleotide probe YₙPₙ, while the reporter oligonucleotide Bₙ hybridizes with the adjacent sequence excerpt Aₙ. If the target nucleotide X_{d} to be tested does not correspond to the probe terminus Yₙ, ligation can not take place, and the same applies when Xₙ is eliminated. Insertion mutants which present an extra nucleotide in Xₙ can still be identified by ligation of YₙPₙ oligonucleotide probes.

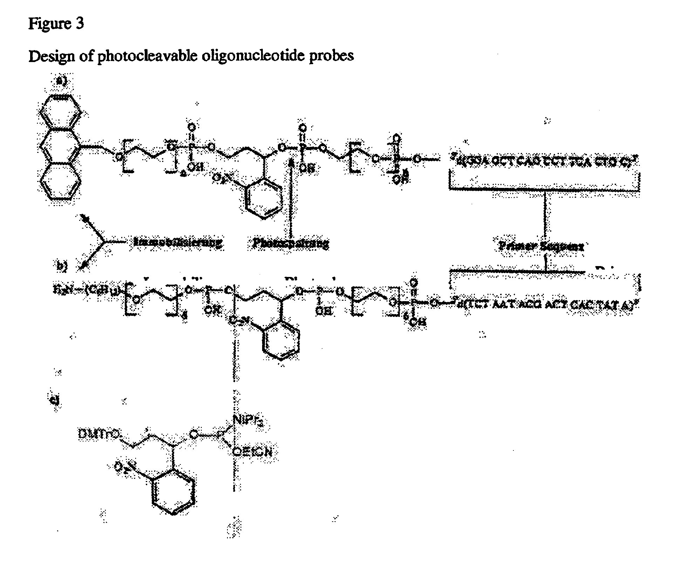

In figure 3, two typical potentially photocleavable oligonucleotide probes are depicted. Both contain a DNA fragment 19 nucleotides long for enzymatic detection by template-dependent modification and an o-nitrobenzyl residue for a targeted photolysis. This is held by flexible hexaethylene or polyethylene glycol spacers, which guarantee unimpeded access for hybridized target sequences or modifying enzymes. Both oligonucleotide probes also contain a functional group which enables immobilization on the solid phase substrate: a) anthracene as a diene in a Diels-Adler reaction with appropriate dienophiles, e.g. maleimide; b) a primary amino group for reaction with suitable active esters such as NHS ester; c) an o-nitrophenyl phosphoramidite residue is shown that can be utilized for generation of a photocleavage site within the photocleavable oligonucleotide probes.

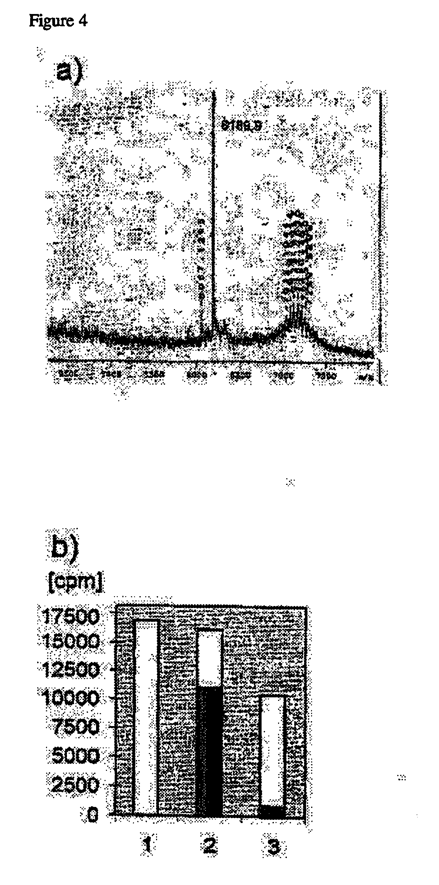

Figure 4a) shows the MALDI TOF mass spectroscopic analysis of the photocleavable oligonucleotide probe from figure 3a). The dominant peak with an m/e of 6189.9 is the signal for the photolytically released oligonucleotide fragment (calculation: [M+H]⁺=6190.0 g/mol). The molecular mass peak of the uncleaved oligonucleotide probe is only visible as trace readings between 7036.2 and 7300.0 g/mol and, due to the polydisperse nature of the polyethyleneglycol spacers, is split up into several peaks. Figure 4b) illustrates the immobilization and photolytic release of photocleavable nucleic acid conjugates: white columns = released nucleic acid; black columns = immobilized nucleic acid; track 1 = inserted, radioactively marked, photocleavable nucleic acid conjugate; track 2 = immobilized fraction after intensive, denaturing washing; track 3 = photolytic release of the immobilized nucleic acid (black columns from track 2).

### Preferred embodyments

The production of the photocleavable oligonucleotide arrays can on the one hand be performed by applying conventionally synthesized oligonucleotide conjugates to appropriately prepared surfaces. The microfluid pipetting or dispensing system required for this is well known to experts in this area. Oligonucleotide probes as required by the invention are mainly fixed on the surface covalently, preferably via a flexible spacer - particularly polyethylene glycol - whereby linking is usually performed at either the 3' or the 5' terminus of the oligonucleotide. The preferably used oligonucleotide conjugates also contain in addition to the photocleavable site another reactive functional group for covalent linking; e.g. biotin, amino, thiol, carboxyl, or diene groups such as anthracene. Suitable photocleavable sites for the invention are chiefly light sensitive o-nitrobenzyl residues, particularly 1-o-nitrophenyl-1,3-propane-diphosphate. A selection of photocleavable oligonucleotide probes is depicted in figure 3.

On the other hand, photocleavable oligonucleotide probes can be synthesized directly on the surface, whereby suitable guidelines for this have been described elsewhere in the literature.

With both manufacturing processes the photocleavable sites can be applied alternatively as a surface-covering layer onto the solid substrate which can then be loaded and synthesized with the desired oligonucleotides at the appropriate positions.

Only two rather complex methods are descibed here as examples, but the detailed description allows the specialist in the field, supported by the knowledge transported by the invention, to easily derive other methods for his analytical task.

### Multiplex mutation analysis by ligation

Firstly, for each mutation point to be analyzed, two external amplification primers and one reporter nucleotide are synthesized by classical solid phase synthesis, whereby in each case one of the amplification primer 5' terminals is derivatized with biotin.

To make the array of photocleavable oligonucleotide probes, a DMTr polyethylene glycol functionalized glass substrate is eroded with trichloracetic acid and then treated over the entire surface with tetrazole and a 0.1M solution of photo-phosphoramidite (figure 3c) in acetonitrile. After repeated washing with acetonitrile, the non-reacted functional groups are blocked with acetic hydride, lutidine and 1-methylimidazole in THF, before treatment with a 1 M iodine solution in pyridine/THF/water. On this o-nitrobenzyl derivatized surface, the corresponding oligonucleotide sequences are synthesized at the appropriate positions using a surface synthesis robot (familiar to experts) in the format depicted in figure 1. Finally, these oligonucleotides are chemically phosphorylated across the entire surface using a 0.1M bis-cyanoethyl-N,N-diisopropyl-phosphoramidite (in CH₃CN) solution. For exposing the photocleavable oligonucleotide probes, the chip is incubated overnight at 55°C in 33% NH₄OH.

The genetic material is isolated from a biological sample of a patient, e.g. a hair root or a drop of blood, using standard procedures. All target sequences are selected by external primer pairs and are amplified using multiplex-PCR in a single reaction mixture. The amplification products are purified on a solid streptavidin-agarose affinity phase using the biotinylated primer. The counterstrands are released into solution by washing in 0.1M NaOH, mixed after neutralization with a set of reporter oligonucleotides, and then applied to the chip (see figure 1). At an end-concentration of 20 mM Tris/HCl (pH=8.3), 50 mM KCl, 10mM MgCl₂, 10mM DTT, 1mM EDTA, 1mM NAD, 0.1% Triton X-100 and 1 U Tth DNA ligase, the ligation reaction is carried out as described in figure 2b) with 20 cycles of 30 sec at 95°C, 1 minute at 50°C and 5 min at 70°C. The chip is then repeatedly washed with 25% DMSO and 0.2M ammonium hydroxide solution and covered finally with a 1 mm thick layer of 3-hydroxypicolinic acid solution which is evaporated to dryness. Using a Nd-YAG laser, the individual points are stimulated one after another at a wavelength of 355 nm and the released oligonucleotide probes (compare figure 4b) are measured by MALDI TOF mass spectrometry as shown in figure 4a). The signals are then evaluated for clinically relevant mutations using integrated software. Every step of the procedure can be automated.

### Multiplex resequencing on a solid phase

For multiplex sequencing of different sequences of a genetic sample, a set of photocleavable oligonucleotide probes is first synthesized by classical solid phase synthesis. They are chosen in such a way that one probe can be hybridized to the target sequence at intervals of 50 nucleotides, whereby the probes for the strand and counterstrand do not differ. The photocleavable oligonucleotide probes are constructed as shown in Figure 3a. The anthracene polyethylene glycol, the hexaethylene glycol spacer as well as the photocleavable o-nitrobenzyl residue are incorporated as corresponding derivatized phosphoramidites. Using the stock solution of these oligonucleotides, more than 10,000 multiplex sequencer reactions to the genetic target sequence can be carried out.

An amino derivatized surface is now functionalized evenly by treatment with 0.1 M maleinimidylhexanoate-NHS ester in DMF over the entire surface. The anthracene functionalized oligonucleotide probes (analogous with 3a) are then pipetted onto this maleinimide surface in 10 nl volumes in an array format using a commercially available gene spotting robot. The chip shown in Figure 1 is freed of non-immobilized oligonucleotide probes by intensive washing after overnight incubation.

A genetic region from a biological sample, containing 250,000 base pairs, is now amplified by cloning and purified by standard procedures. The DNA obtained is distributed amongst four of the above-described chips and mixed with a sequencing mixture (sequenase, dATP, dGTP, dCTP, dTTP as well as each type of ddNTP). After 30 cycles of 15 seconds at 95°C, 15 seconds at 55°C, and 30 seconds at 72°C, the chip is repeatedly washed with 25% DMSO and 0.2M ammonium hydroxide solution and covered finally with a 1 mm thick layer of 3-hydroxypicolinic acid which is then evaporated to dryness. Using a Nd-YAG laser the individual points are stimulated one after another at a wavelength of 355 nm and the released oligonucleotide probes (compare figure 4b) are measured by MALDI TOF mass spectrometry as shown in figure 4a). The measured sequence ladders of the respective terminator nucleotides are evaluated using integrated software, whereby the termination products are checked by measuring their mass and if necessary corrected. After correlation of the strand and counterstrand results, the total sequence obtained is compared with already known data and in this way any deviant mutations can be recorded.

## Claims

1. Method for the analysis of a sample of genetic material for detailed sequence information contained in a large set of distinct sequences of the sample (the "target sequences"), comprising the following steps:
(1) producing an amount of nucleic acid templates containing the target sequences by multiplexed amplification of the sample of genetic material,
(2) using a chip with spatially separated locations containing a photocleavable oligonucleotide probe each for each target sequence to be investigated, the probes covalently bound to the chip surface,
(3) modifying, in a single reaction vessel and by using the templates produced in step (1), all oligonucleotide probes on the chip synchronously in a template-dependent manner so that the information under investigation is transferred from the target sequences of the templates to the probes,
(4) cleaving and mass spectrometrically measuring the spatially separated probes, and
(5) extracting the detailed sequence information from the mass measurements of the probes.

2. The method as in claim 1, wherein the mass of the probes is measured in a time-of-flight mass spectrometer by ionization through laser desorption pulses.

3. The method as in claim 1 or 2, wherein the target sequences are amplified before analysis in a single-vessel reaction.

4. The method as one of the claims 1 to 3, wherein the immobilized probes on the solid substrate are purified from contaminations and released from the template nucleic acid by intensive and, if necessary, denaturing washing after modification.

5. The method as in any of claims 1 to 4, wherein the probes are released from the solid substrate by irradiation after modification and purification and thereby are made accessible for mass spectrometric analysis.

6. The method as in any of the claims 2 to 4, wherein the photolytic cleavage of the oligonucleotide probes from the solid substrate surface occurs simultaneously with their desorption and ionization in the laser desorption pulse.

7. The method as in claim 5 or 6, wherein mass spectrometric detection is performed by MALDI-TOF.

8. The method as in any of the preceding claims wherein the probes are immobilized on a surface suitable for MALDI-TOF spectrometry, the modification of the probes occurs at this surface, and the photolytic release occurs during the MALDI-TOF measurement.

9. The method as in claim 1, wherein the modification of the photocleavable probes occurs by a template-dependent primer elongation.

10. The method as in claim 9, wherein at least one dideoxynucleotide is inserted during the template-dependent primer elongation of the photocleavable probes.

11. The method as in claim 9, wherein the modification of the photocleavable probes occurs by a template-dependent ligation using suitable reporter oligonucleotides.

12. The method as in claim 11, wherein the template specificity of the ligation is raised additionally by the sequence of the reporter oligonucleotides.

13. The method as in claim 12, wherein insertion and deletion mutations are analyzed in particular by the additional template specificity of the reporter oligonucleotides.

14. The method as in any of claims 11 to 13, wherein the reporter oligonucleotides can carry an additional recognition group.

15. The method as in claim 14, wherein the recognition group consists of a mass, fluorescence, or affinity marker, or a photoactive group.

16. The method as in claim 1, wherein the modification of the photocleavable probe is performed by a template-dependent, endonucleolytic cleavage.

17. The method as in claim 16, wherein the endonucleolytic cleavage is performed by restriction enzymes.

18. The method as in claims 16 or 17, wherein methylation patterns of the target sequences can be analyzed by a template-dependent restriction digest of the photocleavable probes.

19. The method as in claim 18, wherein the endonucleolytic cleavage occurs using single-strand specific nucleases.

20. The method as in claims 16 or 19, wherein single strand mismatches of hybridizations between probes and target sequences can be identified by template-dependent nuclease digests of the photocleavable probes.

21. The method as in claim 16, wherein endonucleolytic cleavage occurs using double strand-specific nucleases.

22. The method as in claim 21, wherein the double strand-specific nuclease is RNase H.

23. The method as in claim 1, wherein the photocleavable oligonucleotide probes can contain at least one ribonucleotide.

24. The method as in any of claims 21 to 23, wherein the ribonucleotides of the photocleavable probes can only be template-dependently digested when there is perfect base pairing, leading to detection of the mismatch in the photocleavable probes.

25. The method as in any of claims 9 to 24, wherein the hybridization of the target sequences to the photocleavable oligonucleotide probes and their template-dependent modification can be performed cyclicly a number of times.

26. The method as in claim 25, wherein the enzymes utilized are heat stable and the reaction mixture can be repeatedly warmed directly on the chip.

27. The method as in any of claims 1 to 8, wherein the chip carries, on its surface, 10 to 100,000 spatially separated, photocleavable oligonucleotide probes.

28. The method as in any of claims 1, 5, 6, or 27, wherein the photocleavage site consists of an o-nitrobenzyl residue.

29. The method as in claim 27, wherein the photocleavable oligonucleotide probe is connected additionally to the surface via a spacer in such a way that the enzymatic modification of the probes is facilitated.

30. The method as in any of claims 27 to 29, wherein the probes are immobilized in an array format on the surface of the chip as photocleavable oligonucleotide conjugates.

31. The method as in claim 30, wherein the photocleavable oligonucleotide conjugates carry an additional functional group for immobilization.

32. The method as in claim 31, wherein the additional functional group consists of an amino, sulfhydryl, carboxyl group, biotin, anthracene or a diene.

33. The method as in any of claims 27 to 29, wherein the photocleavable oligonucleotide probe is synthesized directly on the surface.

34. The method as in claim 33, wherein initially the photocleavable sites are synthesized in unison, and then if necessary the spacers are synthesized.

35. Nucleic acid chips with photocleavable oligonucleotide probes as in any of the preceding claims 1 or 27 to 34.

## Patentansprüche

1. Verfahren zur Analyse einer Genmaterialprobe für detaillierte Sequenzinformationen, die in einem großen Satz von unterschiedlichen Sequenzen der Probe (der "Zielsequenzen") enthalten sind, umfassend die folgenden Schritte:
(1) Erzeugen einer Menge von Nukleinsäurematrizen, die die Zielsequenzen enthalten, durch Multiplexverstärkung der Genmaterialprobe,
(2) Verwendung eines Chip mit räumlich getrennten Orten, die jeweils eine photospaltbare Oligonukleotidsonde für jede zu untersuchende Zielsequenz enthalten, wobei die Sonden kovalent mit der Chipoberfläche verbunden sind,
(3) synchrones Modifizieren aller Oligonukleotidsonden auf dem Chip in einer matrizenabhängigen Weise in einem einzigen Reaktionsbehälter durch Verwendung der in Schritt (1) erzeugten Matrizen, so dass die zu untersuchenden Informationen von den Zielsequenzen der Matrizen an die Sonden übertragen werden,
(4) Spalten und massenspektrometrische Messung der räumlich getrennten Sonden und
(5) Entnehmen der detaillierten Sequenzinformationen aus den Massenmessungen der Sonden.

2. Verfahren nach Anspruch 1, wobei die Masse der Sonden in einem Flugzeit-Massenspektrometer durch Ionisieren durch Laserdesorptionsimpulse gemessen wird.

3. Verfahren nach Anspruch 1 oder 2, wobei die Zielsequenzen verstärkt werden bevor sie in einer Reaktion in einem einzigen Behälter analysiert werden.

4. Verfahren nach einem der Ansprüche 1 bis 3, wobei die immobilisierten Sonden auf dem festen Substrat von Verunreinigungen gereinigt werden und von der Nukleinsäure der Matrize durch intensives und gegebenenfalls denaturierendes Waschen nach der Modifikation gelöst werden.

5. Verfahren nach einem der Ansprüche 1 bis 4, wobei die Sonden von dem festen Substrat nach Modifikation und Reinigung durch Bestrahlen gelöst werden und **dadurch** für eine massenspektrometrische Analyse zugänglich gemacht werden.

6. Verfahren nach einem der Ansprüche 2 bis 4, wobei die photolytische Spaltung der Oligonukleotidsonden von der Oberfläche des festen Substrats gleichzeitig mit ihrer Desorption und Ionisieren in dem Laserdesorptionsimpuls stattfindet.

7. Verfahren nach Anspruch 5 oder 6, wobei die massenspektrometrische Detektion durch MALDI-TOF erfolgt.

8. Verfahren nach einem der vorhergehenden Ansprüche, wobei die Sonden auf einer Oberfläche immobilisiert werden, die für MALDI-TOF-Spektrometrie geeignet ist, wobei die Modifikation der Sonden an dieser Oberfläche stattfindet und die photolytische Freisetzung während der MALDI-TOF-Messung stattfindet.

9. Verfahren nach Anspruch 1, wobei die Modifikation der photospaltbaren Sonden durch eine matrizenabhängige Primerverlängerung erfolgt.

10. Verfahren nach Anspruch 9, wobei mindestens ein Dideoxynukleotid während der matrizenabhängigen Primerverlängerung der photospaltbaren Sonden eingeführt wird.

11. Verfahren nach Anspruch 9, wobei die Modifikation der photospaltbaren Sonden durch eine matrizenabhängige Ligation mittels geeigneter Reporter-Oligonukleotide erfolgt.

12. Verfahren nach Anspruch 11, wobei die Matrizenspezifität der Ligation zusätzlich durch die Sequenz der Reporter-Oligonukleotide verstärkt wird.

13. Verfahren nach Anspruch 12, wobei Insertions- und Deletions-Mutationen insbesondere durch die zusätzliche Matrizenspezifität der Reporter-Oligonukleotide analysiert werden.

14. Verfahren nach einem der Ansprüche 11 bis 13, wobei die Reporter-Oligonukleotide eine zusätzliche Erkennungsgruppe tragen können.

15. Verfahren nach Anspruch 14, wobei die Erkennungsgruppe aus einem Masse-, Fluoreszenz- oder Affinitätsmarker oder einer photoaktiven Gruppe besteht.

16. Verfahren nach Anspruch 1, wobei die Modifikation der photospaltbaren Sonde durch eine matrizenabhängige, endonukleolytische Spaltung durchgeführt wird.

17. Verfahren nach Anspruch 16, wobei die endonukleolytische Spaltung durch Restriktionsenzyme ausgeführt wird.

18. Verfahren nach Anspruch 16 oder 17, wobei Methylierungsmuster der Zielsequenzen durch eine matrizenabhängige Restriktionsverdauung der photospaltbaren Sonden analysiert werden können.

19. Verfahren nach Anspruch 18, wobei die endonukleolytische Spaltung mittels einzelstrangspezifischen Nukleasen erfolgt.

20. Verfahren nach Anspruch 16 oder 19, wobei einzelstrangige Diskrepanzen von Hybridisierungen zwischen Sonden und Zielsequenzen durch matrizenabhängige Nukleaseverdauungen der photospaltbaren Sonden identifiziert werden können.

21. Verfahren nach Anspruch 16, wobei endonukleolytische Spaltung mittels doppelstrangspezifischen Nukleasen erfolgt.

22. Verfahren nach Anspruch 21, wobei die doppelstrangspezifische Nuklease RNase H ist.

23. Verfahren nach Anspruch 1, wobei die photospaltbaren Oligonukleotidsonden mindestens ein Ribonukleotid enthalten können.

24. Verfahren nach einem der Ansprüche 21 bis 23, wobei die Ribonukleotide der photospaltbaren Sonden nur matrizenabhängig verdaut werden können, wenn eine perfekte Basenpaarung vorhanden ist, was die Detektion der Diskrepanz in den photospaltbaren Sonden zur Folge hat.

25. Verfahren nach einem der Ansprüche 9 bis 24, wobei die Hybridisierung der Zielsequenzen zu den photospaltbaren Oligonukleotidsonden und ihre matrizenabhängige Modifikation einige Male zyklisch ausgeführt werden kann.

26. Verfahren nach Anspruch 25, wobei die verwendeten Enzyme hitzestabil sind und das Reaktionsgemisch wiederholt direkt auf dem Chip erwärmt werden kann.

27. Verfahren nach einem der Ansprüche 1 bis 8, wobei der Chip auf seiner Oberfläche 10 bis 100000 räumlich getrennte photospaltbare Oligonukleotidsonden trägt.

28. Verfahren nach einem der Ansprüche 1, 5, 6 oder 27, wobei der Ort der Photospaltung aus einem o-Nitrobenzylrest besteht.

29. Verfahren nach Anspruch 27, wobei die photospaltbare Oligonukleotidsonde zusätzlich mit der Oberfläche über einen Abstandshalter so verbunden ist, dass die enzymatische Modifizierung der Sonden ermöglicht wird.

30. Verfahren nach einem der Ansprüche 27 bis 29, wobei die Sonden in einem Arrayformat auf der Oberfläche des Chip als photospaltbare Oligonukleotidkonjugate immobilisiert sind.

31. Verfahren nach Anspruch 30, wobei die photospaltbaren Oligonukleotidkonjugate eine zusätzliche funktionelle Gruppe für die Immobilisierung tragen.

32. Verfahren nach Anspruch 31, wobei die zusätzliche funktionelle Gruppe aus einer Amino-, Sulfhydryl-, Carboxylgruppe, Biotin, Anthrazen oder Dien besteht.

33. Verfahren nach einem der Ansprüche 27 bis 29, wobei die photospaltbare Oligonukleotidsonde direkt auf die Oberfläche synthetisiert wird.

34. Verfahren nach Anspruch 33, wobei die photospaltbaren Orte anfänglich übereinstimmend synthetisiert werden und dann gegebenenfalls die Abstandshalter synthetisiert werden.

35. Nukleinsäurechips mit photospaltbaren Oligonukleotidsonden nach einem der vorhergehenden Ansprüche 1 oder 27 bis 34.

## Revendications

1. Procédé d'analyse d'un échantillon de matériau génétique en ce qui concerne des informations de séquences détaillées contenues dans un grand ensemble de séquences distinctes de l'échantillon (les « séquences cibles »), comprenant les étapes suivantes :
(1) produire une quantité de matrices d'acide nucléique contenant les séquences cibles par amplification multiplexée de l'échantillon de matériau génétique,
(2) utiliser une puce avec des positions espacées dans l'espace contenant une sonde d'oligonucléotide photoclivable, une pour chaque séquence cible devant être étudiée, les sondes étant liées avec une liaison covalente à la surface de puce,
(3) modifier, dans un seul récipient à réaction et en utilisant les matrices produites à l'étape (1), toutes les sondes d'oligonucléotides sur la puce de manière synchrone et dépendant de la matrice de sorte que les informations étudiées soient transférées des séquences cibles des matrices aux sondes,
(4) cliver et soumettre à une mesure par spectrométrie de masse les sondes séparées dans l'espace, et
(5) extraire les informations de séquences détaillées à partir des mesures de masse des sondes.

2. Procédé selon la revendication 1, dans lequel la masse des sondes est mesurée dans un spectromètre de masse à temps de vol par ionisation par des impulsions de désorption laser.

3. Procédé selon la revendication 1 ou 2, dans lequel les séquences cibles sont amplifiées avant analyse dans une seule réaction de récipient.

4. Procédé selon l'une des revendications 1 à 3, dans lequel les sondes immobilisées sur le substrat solide sont purifiées vis-à-vis de contaminations et sont libérées de l'acide nucléique de matrice par un lavage intensif et, si nécessaire, de dénaturation après modification.

5. Procédé selon l'une quelconque des revendications 1 à 4, dans lequel les sondes sont libérées du substrat solide par irradiation après modification et purification et sont ainsi rendues accessibles pour une analyse par spectrométrie de masse.

6. Procédé selon l'une quelconque des revendications 2 à 4, dans lequel le clivage photolytique des sondes d'oligonucléotides par rapport à la surface de substrat solide se produit simultanément à leur désorption et leur ionisation dans l'impulsion de désorption laser.

7. Procédé selon la revendication 5 ou 6, dans lequel la détection par spectrométrie de masse est effectuée par spectrométrie MALDI-TOF.

8. Procédé selon l'une quelconque des revendications précédentes, dans lequel les sondes sont immobilisées sur une surface appropriée pour une spectrométrie MALDI-TOF, la modification des sondes se produit au niveau de cette surface, et la libération photolytique se produit au cours de la mesure MALDI-TOF.

9. Procédé selon la revendication 1, dans lequel la modification des sondes photoclivables se produit par le biais d'un allongement d'amorce dépendant de la matrice.

10. Procédé selon la revendication 9, dans lequel au moins un didésoxynucléotide est inséré au cours de l'allongement d'amorce dépendant de la matrice des sondes photoclivables.

11. Procédé selon la revendication 9, dans lequel la modification des sondes photoclivables se produit par le biais d'une ligature dépendant de la matrice en utilisant des oligonucléotides rapporteurs appropriés.

12. Procédé selon la revendication 11, dans lequel la spécificité de matrice de la ligature est de plus soulevée par la séquence des oligonucléotides rapporteurs.

13. Procédé selon la revendication 12, dans lequel des mutations par insertion et délétion sont analysées en particulier par la spécificité de matrice supplémentaire des oligonucléotides rapporteurs.

14. Procédé selon l'une quelconque des revendications 11 à 13, dans lequel les oligonucléotides rapporteurs peuvent comporter un groupe de reconnaissance supplémentaire.

15. Procédé selon la revendication 14, dans lequel le groupe de reconnaissance est constitué d'un marqueur de masse, de fluorescence, ou d'affinité, ou d'un groupe photoactif.

16. Procédé selon la revendication 1, dans lequel la modification de la sonde photoclivable est effectuée par un clivage endonucléolytique dépendant de la matrice.

17. Procédé selon la revendication 16, dans lequel le clivage endonucléolytique est effectué par le biais d'enzymes de restriction.

18. Procédé selon les revendications 16 ou 17, dans lequel des fragments de méthylation des séquences cibles peuvent être analysés par un condensé de restriction dépendant de la matrice des sondes photoclivables.

19. Procédé selon la revendication 18, dans lequel le clivage endonucléolytique se produit en utilisant des nucléases spécifiques à un seul brin.

20. Procédé selon les revendication 16 ou 19, dans lequel des mésappariements de brin unique d'hybridations entre des sondes et des séquences cibles peuvent être identifiés par le biais de condensés de nucléases dépendant de la matrice des sondes photoclivables.

21. Procédé selon la revendication 16, dans lequel le clivage endonucléolytique se produit en utilisant des nucléases spécifiques à deux brins.

22. Procédé selon la revendication 21, dans lequel la nucléase spécifique à deux brins est la RNase H.

23. Procédé selon la revendication 1, dans lequel les sondes d'oligonucléotides photoclivables peuvent contenir au moins un ribonucléotide.

24. Procédé selon l'une quelconque des revendications 21 à 23, dans lequel les ribonucléotides des sondes photoclivables ne peuvent faire l'objet d'un condensé dépendant de la matrice que lorsqu'il existe un appariement des bases parfait, conduisant à la détection du mésappariement dans les sondes photoclivables.

25. Procédé selon l'une quelconques des revendications 9 à 24, dans lequel l'hybridation des séquences cibles sur les sondes d'oligonucléotides photoclivables et leur modification dépendant de la matrice peuvent être effectuées de manière cyclique un certain nombre de fois.

26. Procédé selon la revendication 25, dans lequel les enzymes utilisées sont thermiquement stables et le mélange de réaction peut être réchauffé de manière répétée directement sur la puce.

27. Procédé selon l'une quelconques des revendications 1 à 8, dans lequel la puce supporte, sur sa surface, de 10 à 100 000 sondes d'oligonucléotides photoclivables, séparées dans l'espace.

28. Procédé selon l'une quelconques des revendications 1, 5, 6 ou 27, dans lequel le site de photoclivage est constitué d'un résidu de o-nitrobenzyle.

29. Procédé selon la revendication 27, dans lequel la sonde d'oligonucléotide photoclivable est de plus reliée à la surface par le biais d'un élément d'espacement de sorte que la modification enzymatique des sondes soit facilitée.

30. Procédé selon l'une quelconques des revendications 27 à 29, dans lequel les sondes sont immobilisées dans un format de réseau sur la surface de la puce comme des conjugués d'oligonucléotides photoclivables.

31. Procédé selon la revendication 30, dans lequel les conjugués d'oligonucléotides photoclivables comportent un groupe fonctionnel supplémentaire pour l'immobilisation.

32. Procédé selon la revendication 31, dans lequel le groupe fonctionnel supplémentaire est constitué d'un groupe amine, sulfhydryle, carboxylique, de la biotine, de l'anthracène ou d'un diène.

33. Procédé selon l'une quelconques des revendications 27 à 29, dans lequel la sonde d'oligonucléotide photoclivable est synthétisée directement sur la surface.

34. Procédé selon la revendication 33, dans lequel, initialement, les sites photoclivables sont synthétisés à l'unisson, et ensuite si nécessaire les éléments d'espacement sont synthétisés.

35. Puces d'acides nucléiques avec des sondes d'oligonucléotides photoclivables selon l'une quelconque des revendications précédentes 1 ou 27 à 34.
